# EUROPEAN PATENT APPLICATION

(11) **EP 4 736 875 A1**
(43) Date of publication of application: **06.05.2026**
(21) Application number: 24941298.2
(22) Date of filing: 02.08.2024
(51) Int. Cl.: A61K 45/00, C12Q 1/02, A61P 37/04, A61P 35/00, A61P 31/00

(54) **USE OF RAGE RECEPTOR INHIBITOR IN PREPARATION OF DRUG FOR INHIBITING LYMPHOCYTE REDUCTION OR DEATH**

(30) Priority: 31.05.2024 CN 202410696728
(71) Applicant: The Third Xiangya Hospital of Central South University, Changsha, Hunan 410013 (CN)
(72) Inventor: LV, Ben, Changsha, Hunan 410013 (CN); LI, Yi, Changsha, Hunan 410013 (CN); TANG, Yiting, Changsha, Hunan 410013 (CN)
(74) Representative: impuls legal PartG mbB
(86) International application number: PCT/CN2024/109517
(87) International publication number: WO 2025/246020

(57) **Abstract**

Disclosed is use of a Rage receptor inhibitor in the preparation of a drug for inhibiting lymphocyte reduction or death. Using Rage as a target for in vitro screening of the drug for inhibiting lymphocyte reduction or death provides a new research direction for preparing more efficient tumor drugs.

## Description

### TECHNICAL FIELD

The present disclosure belongs to the field of biotechnology, and specifically relates to use of a receptor for advanced glycation endproducts (RAGE) inhibitor in preparation of a medicament for inhibiting lymphocyte reduction or death.

### BACKGROUND

The incidence and mortality of malignant tumors and severe infections are on the rise, making them the second and third leading causes of death worldwide, respectively. A common feature of malignant tumors and severe infections is lymphocyte depletion, which leads to persistent immunosuppression or tumor immune escape in the body. Reducing lymphocyte depletion, enhancing lymphocyte viability and promoting immune response of the body can improve the overall survival rate. Since immunotherapy can enhance immunocompetence of the body and has potentially high specificity and low side effects, it demonstrates a broad development prospect.

Immune checkpoint inhibitors (ICls) exert their effects by blocking the immunosuppressive ligand-receptor interaction involving CTLA-4 and PD-1. Tumor immunotherapy centered on immune checkpoint blockade (ICB) has revolutionized the landscape of tumor treatment. Although ICB therapy attenuates the primary inhibitory signals for T lymphocytes and enhances potential T cell-mediated antitumor activity, it is accompanied by progressive exhaustion and depletion-induced death of tumor-reactive T cells, leading to widespread drug resistance and failure to exert durable efficacy, thereby influencing treatment outcomes.

To achieve optimal clinical outcomes, it is necessary to enhance response rate and duration of the body's immune function. Accordingly, specific small molecule inhibitors hold promise not only for suppressing key oncogenic signaling pathways, but also for sustaining lymphocyte viability while enhancing their response to exert more long-lasting effect, thereby serving as adjuvant agents to existing ICIs. Current ICB therapy benefits only a minority of cancer patients, and many fail to derive long-term benefits from treatment. Consequently, there is an urgent need in the scientific community to identify a mechanism capable of enhancing immune cell viability and preventing immune cell depletion to alleviate immunosuppression. This will facilitate the development of ICIs against novel targets, the combination of ICIs targeting different pathways, and the integration of ICIs with other treatment modalities, ultimately improving immune cell viability and reducing immune cell exhaustion.

### SUMMARY

The present disclosure aims to at least resolve one of the technical problems mentioned above existing in the prior art. To this end, the present disclosure proposes use of RAGE as a target for *in vitro* screening of products for inhibiting lymphocyte reduction or death.

The present disclosure further proposes use of a RAGE inhibitor in preparation of a product for inhibiting lymphocyte reduction or death.

The present disclosure further proposes a method for screening a medicament that inhibits lymphocyte reduction or death.

According to a first aspect of the present disclosure, use of RAGE as a target for *in vitro* screening of products for inhibiting lymphocyte reduction or death is provided.

In some embodiments of the present disclosure, the lymphocytes comprise at least one of T lymphocytes and B lymphocytes.

According to a second aspect of the present disclosure, use of a RAGE inhibitor in preparation of a product for inhibiting lymphocyte reduction or death is provided.

In some embodiments of the present disclosure, the RAGE inhibitor is a molecule capable of inhibiting transcription or translation of a RAGE gene, or a molecule capable of specifically inhibiting expression or activation of a RAGE protein.

In some embodiments of the present disclosure, the RAGE inhibitor is a nucleic acid molecule, an antibody, or a small-molecule compound.

In some embodiments of the present disclosure, the nucleic acid molecule is siRNA, shRNA, or sgRNA.

In some embodiments of the present disclosure, the small-molecule compound is one or two selected from the group consisting of FPS-ZM1 and Azeliragon.

In some embodiments of the present disclosure, the product for inhibiting lymphocyte reduction or death has a function of any one of the following (1)-(3):
(1) increasing the number of CD3⁺ T cells;
(2) increasing the number of CD19⁺ B cells; and
(3) inhibiting programmed cell death.

In some embodiments of the present disclosure, the programmed cell death is pyroptosis, apoptosis, or necroptosis.

In some embodiments of the present disclosure, the RAGE inhibitor can be used alone or can be used in combination with other therapeutic agents.

In some embodiments of the present disclosure, the product for inhibiting lymphocyte reduction or death includes the RAGE inhibitor alone or a composition of the RAGE inhibitor and other therapeutic agents.

In some embodiments of the present disclosure, the RAGE inhibitor is formulated into a pharmaceutical composition with a pharmaceutically acceptable excipient.

In some embodiments of the present disclosure, the pharmaceutically acceptable excipient is one or more selected from the group consisting of a carrier, a diluent, a binder, a lubricant, and a wetting agent.

In some embodiments of the present disclosure, the pharmaceutical composition is in one or more forms selected from the group consisting of a solution, an injection, a spray, a nasal drop, an aerosol, a dry powder inhaler, a tablet, a capsule, and granules.

In some embodiments of the present disclosure, the pharmaceutical composition can be administered into a body such as muscle, intradermal, subcutaneous, venous or mucosal tissues by means of injection, spraying, nasal drops, eye drops, permeation, absorption, or physically or chemically-mediated methods, or is administered into a body after being mixed with or encapsulated by another substance.

According to a third aspect of the present disclosure, a method for screening a medicament that inhibits lymphocyte reduction or death is provided, including: identifying substances capable of inhibiting or blocking the expression and/or function of RAGE as candidate medicaments by using RAGE as a medicament target.

In some embodiments of the present disclosure, the method includes: treating cells with a candidate medicament *in vitro* and incubating, or administering a candidate medicament to an animal model *in vivo*; and subsequently assessing the reduction or death of lymphocytes in the cells or the animal model.

In some embodiments of the present disclosure, the cells may be derived from a mammal.

Whether a candidate medicament is therapeutically effective can be determined by assessing reduction or death of lymphocytes after incubation. Generally, a medicament that inhibits the reduction or death of lymphocytes by 50%, 60%, 70%, 80%, 90%, 95%, 99% or even 100% compared to a control can be determined to be therapeutically effective.

In the present disclosure, "treatment" means slowing, interruption, arrest, control, stopping, alleviation or reversal of the progression or severity of a sign, symptom, disorder, condition, or disease after its onset, but does not necessarily entail the complete elimination of all disease-related signs, symptoms, conditions or disorders.

In the present disclosure, "prevention" means inhibiting the occurrence of, or delaying the onset of, a symptom or disease by administration of the product described herein.

In some embodiments of the present disclosure, a medicament is determined to be therapeutically effective if it can reduce lymphocyte reduction or death by at least 50%.

According to some embodiments of the present disclosure, at least the following beneficial effects are achieved: the present disclosure creatively uses RAGE as a target for *in vitro* screening of medicaments for inhibiting lymphocyte reduction or death, providing a new research direction for preparation of more effective antitumor medicaments.

### BRIEF DESCRIPTION OF DRAWINGS

The present disclosure is further described below in conjunction with the accompanying drawings and embodiments, in which:
FIG. 1 is a schematic diagram illustrating the experimental procedure in Example 1 of the present disclosure.

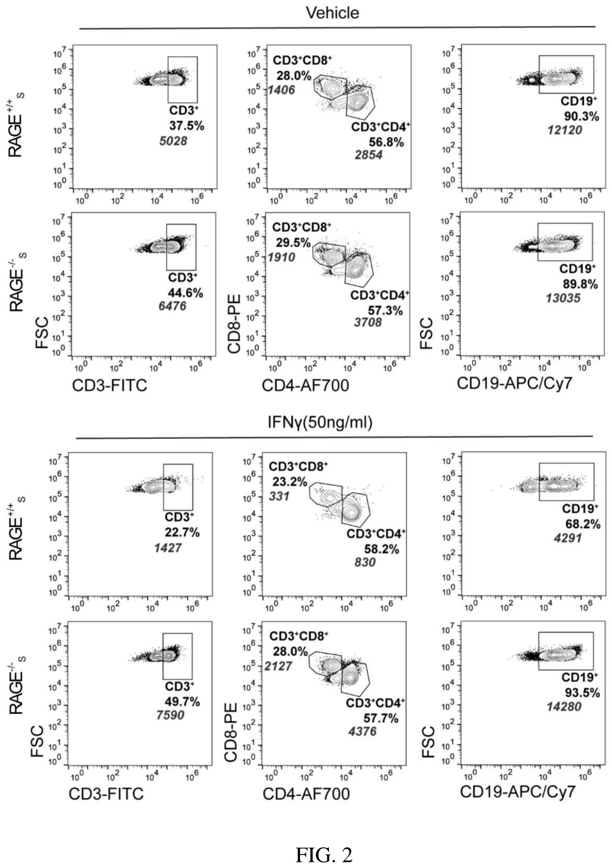

FIG. 2 shows flow cytometry plots illustrating survival rates and cell counts of T and B lymphocytes in upper-chamber RAGE^{+/+} or RAGE^{-/-} splenocytes from Example 1 of the present disclosure, wherein CD3-FITC, CD4-AF700 and CD8-PE are used to identify T lymphocytes, and CD19-APC/Cy7 is used to identify B lymphocytes; RAGE^{+/+}_{S} and RAGE^{-/-}_{S} denote upper-chamber splenocytes of respective genotypes co-cultured for 72 h with lower-chamber RAGE^{+/+}_{PM}; Vehicle represents controls treated with an equal volume of vehicle, IFNγ (50 ng/mL) denotes experimental groups; and red numerals indicate cell counts corresponding to respective percentages of lymphocytes.

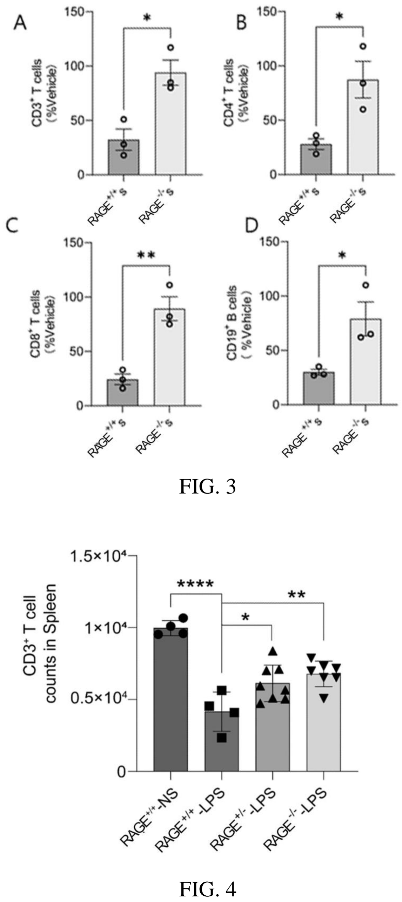

FIG. 3 are bar graphs showing T and B lymphocytes in upper-chamber splenocytes from Example 1 of the present disclosure, corresponding to the flow cytometry results of FIG.2, wherein %Vehicle represents the ratio of lymphocyte counts in RAGE^{+/+}_{S} and RAGE^{-/-}_{S} experimental groups to the lymphocyte counts in their respective controls; Panel A shows a ratio of CD3⁺ T lymphocyte counts in the RAGE^{+/+}_{S} and RAGE^{-/-}_{S} experimental groups to the lymphocyte counts in their respective controls; Panel B shows a ratio of CD4⁺ T lymphocyte counts in RAGE^{+/+}_{S} and RAGE^{-/-}_{S} experimental groups to the lymphocyte counts in their respective controls; Panel C shows a ratio of CD8⁺ T lymphocyte counts in the RAGE⁺⁺_{S} and RAGE^{-/-}_{S} experimental groups to the lymphocyte counts in their respective controls; Panel D shows a ratio of CD19⁺ B lymphocyte counts in the RAGE^{+/+}_{S} and RAGE^{-/-}_{S} experimental groups to the lymphocyte counts in their respective controls, where * denotes p < 0.05, and ** denotes p < 0.01.
FIG. 4 is a bar graph showing CD3⁺ T lymphocyte counts measured by flow cytometry in the endotoxemia model of Example 2 of the present disclosure, wherein *denotes p < 0.05, ** denotes p < 0.01, and **** denotes p < 0.0001.
FIG. 5 is a bar graph showing CD4⁺ T lymphocyte counts measured by flow cytometry in the endotoxemia model of Example 2 of the present disclosure, wherein ** denotes p < 0.01 and **** denotes p < 0.0001.
FIG. 6 is a bar graph showing CD8⁺ T lymphocyte counts measured by flow cytometry in the endotoxemia model of Example 2 of the present disclosure, wherein * denotes p < 0.05, ** denotes p < 0.01, and *** denotes p < 0.001.
FIG. 7 shows immunohistochemical staining of CD3⁺ T lymphocytes in spleen tissue from the endotoxemia model of Example 2 of the present disclosure.
FIG. 8 is a bar graph showing CD3⁺ T lymphocyte percentages measured by flow cytometry in the cecal ligation and puncture model of Example 2 of the present disclosure, wherein ** denotes p < 0.01, *** denotes p < 0.001, and **** denotes p < 0.0001.
FIG. 9 is a bar graph showing CD4⁺ T lymphocyte percentage measured by flow cytometry in the cecal ligation and puncture model of Example 2 of the present disclosure, wherein ** denotes p < 0.01, *** denotes p < 0.001, and **** denotes p < 0.0001.
FIG. 10 is a bar graph showing CD8⁺ T lymphocyte percentages measured by flow cytometry in the cecal ligation and puncture model of Example 2 of the present disclosure, wherein ** denotes p < 0.05, and ** denotes p < 0.01.
FIG. 11 is a bar graph showing CD19⁺ B lymphocyte percentages measured by flow cytometry in the cecal ligation and puncture model of Example 2 of the present disclosure, wherein *** denotes p < 0.001 and **** denotes p < 0.0001.
FIG. 12 shows Western Blot detection results of programmed cell death of the cells in Example 2.

### DETAILED DESCRIPTION

The concept and technical effects of the present disclosure will be clearly and completely described below in conjunction with the examples, so as to enable a full understanding of the purpose, features, and effects of the present disclosure. Obviously, the described examples are only a part of the examples of the present disclosure, rather than all the examples. Based on the examples of the present disclosure, other examples obtained by those skilled in the art without creative efforts shall all fall within the protection scope of the present disclosure.

Experimental Materials: RAGE^{-/-} refers to RAGE gene knockout mice on a C57 background, and RAGE^{+/+} and RAGE^{+/-} refer to wild-type littermate control mice of the RAGE gene knockout mice. All mice were generously provided by Southern Medical University. Detailed methods for the generation and identification of the mice are described in the reference "*RAGE Control of Diabetic Nephropathy in a Mouse Model: Effects of RAGE Gene Disruption and Administration of Low-Molecular Weight Heparin*". The mice used in the experiments were 8-12 weeks old.

### Example 1 Use of a RAGE inhibitor in preparation of a product for inhibiting lymphocyte reduction or death.

This example provided the use of a RAGE inhibitor in preparation of a product for inhibiting lymphocyte reduction or death. Specifically, RAGE knockout mice were used for the subsequent experimental verification.

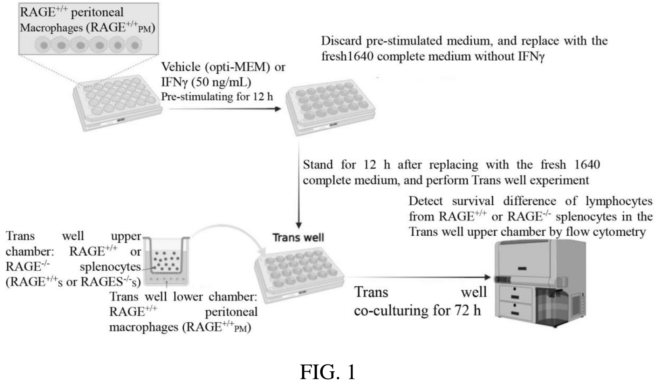

Experimental Method: The procedure is schematically shown in FIG.1 and briefly described as follows: pre-stimulating RAGE^{+/+} peritoneal macrophages (RAGE^{+/+}_{PM}) in a Transwell lower chamber with IFNγ at a final concentration of 50 ng/mL or an equal volume of Opti-MEM (Vehicle), after 12 h, removing the medium in the lower chamber, replacing with an equal volume of RPMI-1640 complete medium, culturing for additional 12 h, adding splenocytes, namely RAGE^{+/+} or RAGE^{-/-} splenocytes (RAGE^{+/+}_{S} or RAGE^{-/-}_{S}), to a Transwell upper chamber, co-culturing Transwell upper-chamber and Transwell lower-chamber cells for 72 h, determining survival rates and counts of various lymphocytes in upper-chamber RAGE^{+/+} or RAGE^{-/-} splenocytes by flow cytometry. Specific steps were as follows:

### (1) Extracting peritoneal macrophages from wild-type mice:

### 1) Preparation:

Intraperitoneally injecting 3% thioglycolate broth into 8-12-week-old RAGE⁺/⁺ mice (10-week-old mice in this example), specifically including: disinfecting an injection site and surrounding area (the injection site was selected at a midpoint between an abdomen midline and a groin connecting line in principle, where fewer abdominal organs were present, thereby reducing puncture risk), holding a syringe (containing 2.5 mL of 3% thioglycolate broth) in the right hand and with the bevel facing upwardly, slowly inserting the needle at a certain angle for 2 mm , and then performing Z-track needle insertion to penetrate the peritoneal cavity at a 45° angle to the abdomen until a distinct loss of resistance was felt, aspirating to confirm absence of blood return, slowly infusing the thioglycolate broth into the peritoneal cavity, withdrawing the needle, returning the mice to cage, and observing conditions of the mice. The mice may die immediately if the thioglycolate broth was injected into the blood vessel.

2) Cell extraction: After 3 days, sacrificing the mice by cervical dislocation, completely immersing the mice in ethanol for 5 minutes for disinfection, drawing up 15 mL of RPMI-1640 medium by a 20 mL syringe, turning on a laminar flow hood, transferring the mice on sterile absorbent paper inside the hood in a supine position and with heads oriented to the left, gently lifting the mid-lower abdominal skin with curved forceps, making a 2-3 mm incision along the forceps by ophthalmic scissors, carefully tearing the skin to fully expose the abdominal cavity, lifting the peritoneum with curved forceps in the left hand, inserting the 20 mL syringe pre-filled with the RPMI-1640 medium and held in the right hand into the peritoneal cavity, immediately injecting the RPMI-1640 medium to dislodge the omentum and intestines adhering to the abdominal wall, maintaining the needle bevel facing the operator, performing peritoneal lavage repeatedly for 5-8 times until the RPMI-1640 medium was observed visibly turbid, and aspirating the peritoneal lavage fluid, and collecting into 50 mL sterile centrifuge tubes.

3) Cell counting and plating: Transferring all cells to the laminar flow hood, filtering by a cell strainer to remove adipose tissue from the lavage fluid to obtain a cell suspension, centrifuging at 129 × g for 5 min at 4 °C, discarding the supernatant, adding 2 mL of pre-warmed RPMI-1640 complete medium, repeatedly pipetting up and down for approximately 50 times, transferring 10 µL of the cell suspension into 390 µL of the RPMI-1640 complete medium and mixing thoroughly, loading 10 µL to a hemocytometer by a micropipette, and counting under a microscope.

A total number of cells in two diagonally opposite large squares on the hemocytometer was counted, an average value was calculated, and the number of cells per milliliter of the cell suspension was determined as a mean count of the cells in two large squares × 10⁴.

Cell plating: after counting, transferring the cells into respective culture plates based on cell counting results, wherein 24-well plates were used for experiment with 500 µL cell suspension per well at a cell density of 5 × 10⁵ cells per 500 µL of RPMI-1640 complete medium, and the RPMI-1640 complete medium was composed of RPMI-1640 supplemented with 10% heat-inactivated fetal bovine serum, 1% penicillin/streptomycin, and 10 µmol/L β-mercaptoethanol.

4) Pre-stimulating RAGE⁺/⁺ peritoneal macrophages with recombinant mouse IFNγ at a final concentration of 50 ng/mL, discarding the cell culture supernatant after 12 h, replacing with fresh RPMI-1640 complete medium, returning to the cell culture incubator for additional 12-hour culture, seeding the thus-treated RAGE⁺/⁺ peritoneal macrophages (RAGE^{+/-} _{PM}) into the Transwell lower chamber. For the Vehicle control, RAGE⁺/⁺ peritoneal macrophages were pre-stimulated with an equal volume of Opti-MEM instead of the recombinant IFNγ.

### (2) Extracting splenocytes from RAGE⁻/⁻ mice and RAGE⁺/⁺ mice:

1) harvesting spleens from RAGE⁻/⁻ mice and RAGE⁺/⁺ mice which were matched with RAGE⁻/⁻ mice in age and sex, and preparing single-cell splenocyte suspensions by passing through a 40 µm strainer;
2) centrifuging the single-cell splenocyte suspensions for cell pellet at 500 × g and 4 °C for 5 min;
3) removing supernatant, adding 1 × red blood cell lysis buffer (5 mL/spleen), and lysing red blood cells at room temperature for 5-8 min;
4) washing cells with 5 mL of ice-cold 1 × PBS, followed by centrifugation at 4 °C and 500 × g for 5 min, and repeating for three times;
5) discarding PBS after washing, and resuspending the cell pellet in 2 mL of RPMI-1640 complete medium which was composed of RPMI-1640 supplemented with 10% heat-inactivated fetal bovine serum, 1% penicillin/streptomycin and 10 µmol/L β-mercaptoethanol;
6) filtering the resuspended cells through a 40 µm strainer;
7) counting the cells, and assessing cell viability with 0.4% trypan blue while ensuring cell survival rate ≥85% for subsequent operations;
8) seeding the RAGE⁺/⁺ peritoneal macrophages extracted and treated in the step (1) into the Transwell lower chambers, respectively adding splenocytes from either RAGE⁻/⁻ mice or RAGE⁺/⁺ mice to Transwell upper chambers (5×10⁵ cells/50 µL per well), and co-culturing for 72 h; and
9) collecting splenocytes from Transwell upper chambers, staining with CD3-FITC, CD4-AF700, CD8-PE, and CD19-APC/Cy7, and assessing the survival rates of T and B lymphocytes in splenocytes from RAGE⁻/⁻ mice or RAGE⁺/⁺ mice by flow cytometry.

As shown in FIGS. 2-3, under the same treatment condition, both the survival rates and counts of T and B lymphocytes in the RAGE^{-/-}_{S} group are significantly higher than those in the RAGE^{+/-}_{S} group, indicating that RAGE gene knockout can inhibit depletion of T and B lymphocytes. The RAGE target screened in the present disclosure can be used to maintain the viability of T and B lymphocytes and inhibit their depletion. The screening method in the present disclosure can be effectively used for screening medicaments that inhibits the depletion of T and B lymphocytes.

### Example 2 Use of a RAGE inhibitor in preparation of an agent for inhibiting programmed cell death

Through *in vivo* animal experiments, this example demonstrated that blocking RAGE can inhibit programmed cell death such as pyroptosis, apoptosis, and necroptosis. Specific experimental steps were as follows:

### 1. Experiment demonstrating inhibition on lymphocyte reduction or death

### (1) In vivo animal experiments: endotoxemia mouse models

1) Experiment grouping: RAGE⁺/⁺-NS group, RAGE⁺/⁺-LPS group, RAGE⁺/⁻-LPS group and RAGE⁻/⁻-LPS group, wherein LPS denotes intraperitoneal injection of 8 mg/kg lipopolysaccharide (LPS), and NS (Normal Saline) denotes intraperitoneal injection of normal saline in a volume equal to that of LPS; and RAGE⁺/⁺ denotes wild-type mice; RAGE⁺/⁻denotes mice heterozygous for RAGE knockout; and RAGE⁻/⁻ denotes mice homozygous for RAGE knockout. Each group included at least 3 mice aged 8-12 weeks, wherein 10-week-old mice were used in this example.
2) Weighing the mice, intraperitoneally injecting 100 µL of normal saline into the peritoneal cavities of mice in a control group, respectively, and intraperitoneally injecting LPS in an equal volume to normal saline at a dose of 8 mg/kg into the mice of the RAGE⁺/⁺-LPS, RAGE⁺/⁻-LPS, and RAGE⁻/⁻-LPS groups, respectively.
3) At 72 h post-modeling, harvesting spleens from mice by the same procedure as in Example 1, grinding to prepare single-cell splenocyte suspensions, and analyzing the viability of lymphocytes.

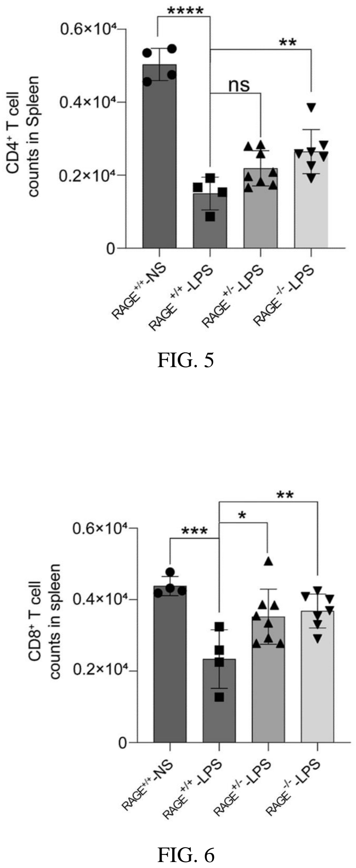

The results of the endotoxemia mouse model are shown in FIGS. 4-7. As can be seen, the numbers of viable T and B lymphocytes in the RAGE⁻/⁻ and RAGE⁺/⁻ groups receiving intraperitoneal LPS injection are significantly higher than those in the RAGE⁺/⁺ group, indicating that RAGE knockout or knockdown can inhibit the depletion of T and B lymphocytes.

### (2) in vivo animal experiments: cecal ligation and puncture (CLP) mouse models

1) Experiment grouping: RAGE^{+/+}-Sham group, RAGE^{-/-}-Sham group, RAGE^{+/+}-CLP group and RAGE^{-/-}-CLP group, wherein Sham denotes a sham-operated group; and CLP denotes a cecal ligation and puncture-operated group.
2) CLP modeling including:
   ① anesthetizing mice with 1% pentobarbital sodium according to body weight, and shaving the abdominal hair of mice in each group by a hair clipper;
   ② securing the limbs of mice in a supine position, and disinfecting the abdomen with ethanol and iodophor;
   ③ incising the skin along a lower midline of the abdomen to create a longitudinal incision of less than 1 cm, and bluntly dissecting between the skin and rectus abdominis using scissors;
   ④ cutting through the rectus abdominis to expose abdominal contents;
   ⑤ lifting the left abdominal skin and rectus abdominis using curved forceps, gently inserting straight forceps for locating the cecum, and carefully exteriorizing the cecum onto gauze;
   ⑥ Ligating the cecum: ligating the cecum at its distal end, approximately at 35% of its total length;
   ⑦ performing cecal puncturing: confirming whether feces were present at the distal end of the cecum or not, if not, gently pushing cecal contents from a proximal end toward the distal end, creating a single puncture at the distal end of the cecum using a needle of a 10 mL syringe while avoiding blood vessels, extruding a small amount fecal contents through the puncture, wiping off using a cotton swab, and extruding feces at approximately the size of a sesame seed;
   ⑧ closing the abdominal cavity: lifting right abdominal muscle and right skin, returning the cecum with straight forceps while maintaining the right side exposed as much as possible and avoiding fecal contamination of the incision;
   ⑨ performing postoperative fluid resuscitation: subcutaneously injecting 37°C pre-heated normal saline (5 mL/100 g, approximately 1 mL/mouse in this example) into the bilateral dorsal regions, and placing mice on a heating pad for thermal insulation until they awaken indicates successful modeling; and
   ⑩ 7 days post-modeling, harvesting spleens from mice using the same procedure as in Example 1, grinding to prepare single-cell splenocyte suspensions, and analyzing lymphocyte death.

Note: The sham-operated group underwent an identical surgical procedure, except that the cecum was neither ligated nor punctured.

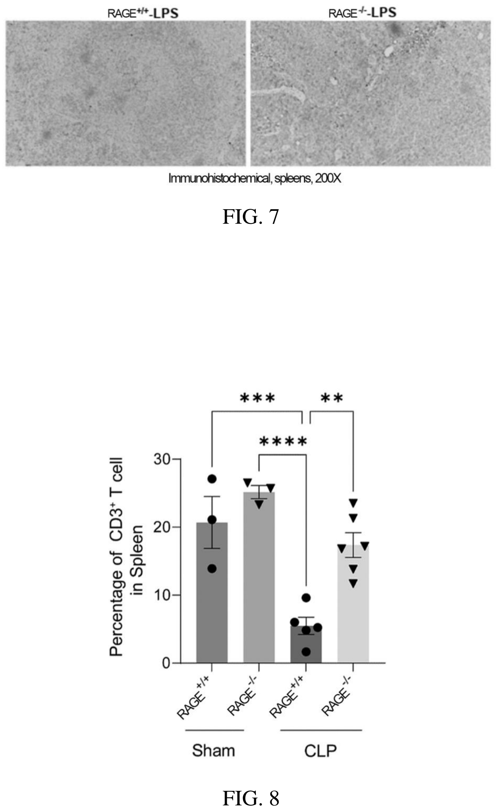

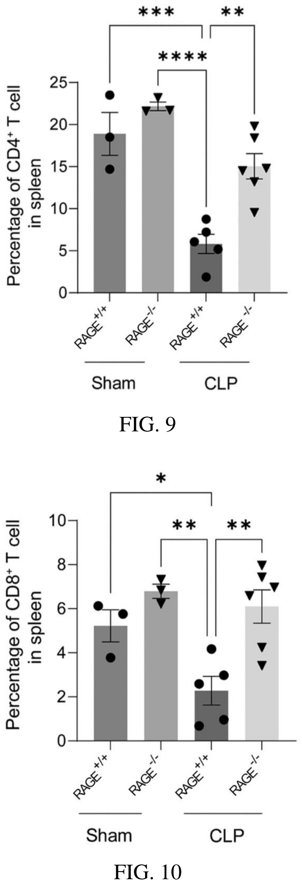

The results of the CLP models are shown in FIGS. 8-11. As can be seen, the survival rates of T and B lymphocytes in the RAGE⁻/⁻ group in the CLP model are significantly higher than those in the RAGE⁺/⁺ group, indicating that RAGE blocking can significantly reduce death of T and B lymphocytes.

### 2. Experiment demonstrating that RAGE blocking can be used for inhibiting programmed cell death

Western Blot analysis was performed to assess pyroptosis, apoptosis and necroptosis in splenocytes from endotoxemia animal models (RAGE⁺/⁺ and RAGE⁻/⁻ endotoxemia animal models) after RAGE blocking.

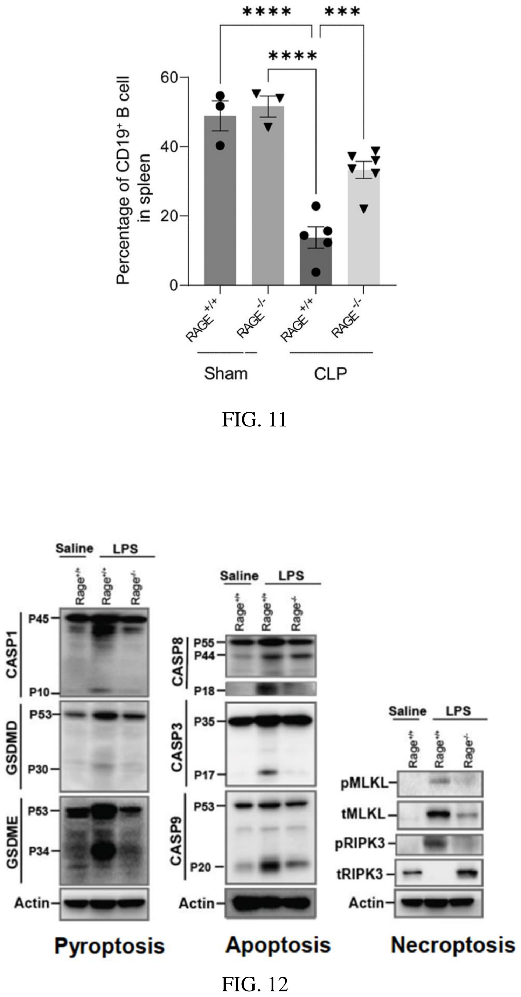

The Western Blot results are shown in FIG. 12. As can be seen, RAGE blocking can significantly reduce programmed cell death, including pyroptosis, apoptosis, and necroptosis.

The examples of the present disclosure have been described in detail above with reference to the accompanying drawings. However, the present disclosure is not limited to the above-described examples, and various modifications may be made without departing from the protection scope of the present disclosure within the scope of knowledge possessed by those having ordinary skills in the art. In addition, the examples of the present disclosure and the features in the examples may be combined with each other without conflict.

## Claims

1. Use of RAGE as a target for *in vitro* screening of products for inhibiting lymphocyte reduction or death.

2. The use according to claim 1, wherein the lymphocytes comprise T lymphocytes and B lymphocytes.

3. Use of a RAGE inhibitor in preparation of a product for inhibiting lymphocyte reduction or death.

4. The use according to claim 3, wherein the RAGE inhibitor is a molecule capable of inhibiting transcription or translation of a RAGE gene, or a molecule capable of specifically inhibiting expression or activation of a RAGE protein;
preferably, the RAGE inhibitor is a nucleic acid molecule, an antibody, or a small-molecule compound;
more preferably, the nucleic acid molecule is siRNA, shRNA, or sgRNA;
more preferably, the small-molecule compound is one or two selected from the group consisting of FPS-ZM1 and Azeliragon.

5. The use according to claim 3, wherein the product for inhibiting lymphocyte reduction or death has a function of any one of the following (1)-(3):
(1) increasing the number of CD3⁺ T cells;
(2) increasing the number of CD19⁺ B cells; and
(3) inhibiting programmed cell death;
preferably, the programmed cell death is pyroptosis, apoptosis, or necroptosis.

6. The use according to claim 3, wherein the product for inhibiting lymphocyte reduction or death comprises the RAGE inhibitor alone or a composition of the RAGE inhibitor and other therapeutic agents.

7. The use according to claim 3, wherein the RAGE inhibitor is formulated into a pharmaceutical composition with a pharmaceutically acceptable excipient; preferably, the pharmaceutically acceptable excipient is one or more selected from the group consisting of a carrier, a diluent, a binder, a lubricant, and a wetting agent.

8. The use according to claim 7, wherein the pharmaceutical composition is in one or more forms selected from the group consisting of a solution, an injection, a spray, a nasal drop, an aerosol, a dry powder inhaler, a tablet, a capsule, and granules.

9. A method for screening a medicament that inhibits lymphocyte reduction or death, comprising: identifying substances capable of inhibiting or blocking the expression and/or function of RAGE as candidate medicaments by using RAGE as a medicament target.

10. The method according to claim 9, comprising: treating cells with a candidate medicament *in vitro* and incubating, or administering a candidate medicament to an animal model *in vivo*; and subsequently assessing the reduction or death of lymphocytes in the cells or the animal model.
